# EUROPEAN PATENT SPECIFICATION

(11) **EP 4 559 445 B1**
(45) Date of publication and mention of the grant of the patent: **11.03.2026**
(21) Application number: 24213496.3
(22) Date of filing: 18.11.2024
(51) Int. Cl.: A61F 13/15, A61F 13/514, B29C 55/08, B29C 55/18

(54) **APPARATUS AND METHOD FOR MAKING AN ABSORBENT SANITARY ARTICLE**
VORRICHTUNG UND VERFAHREN ZUR HERSTELLUNG EINES SAUGFÄHIGEN HYGIENEARTIKELS
APPAREIL ET PROCÉDÉ DE FABRICATION D'UN ARTICLE HYGIÉNIQUE ABSORBANT

(30) Priority: 22.11.2023 IT 202300024813
(43) Date of publication of application: 28.05.2025
(73) Proprietor: GDM S.p.A., 40133 Bologna (IT)
(72) Inventor: ZAVALLONI, Alessandro, I-40133 Bologna (BO) (IT); TOSCANI, Federico, I-40133 Bologna (BO) (IT); ROSSI, Eros, I-40133 Bologna (BO) (IT)
(74) Representative: Porta & Consulenti Associati S.p.A.

(56) References cited:
- WO-A1-2011/025032
- WO-A1-2013/137817
- WO-A1-99/02114
- JP-B2- 5 647 846

## Description

The present invention relates to an apparatus and method for making an absorbent sanitary article.

Absorbent sanitary items are for example female sanitary napkins, baby nappies, nappies for people suffering from incontinence and the like. In the following description, reference will be made to the non-limiting example of female sanitary napkins.

Typically, a female sanitary napkin comprises a top and a bottom layer, called the "topsheet" and "backsheet" respectively in industry jargon, superimposed and joined together. An absorbent core is arranged between the top layer and the bottom layer.

The top layer is made of a material permeable to body fluids and is configured to face the user's body, in contact with it, when the sanitary napkin is applied to the undergarment.

The absorbent core has the function of absorbing the body fluids that pass through the top layer and of retaining them without leaks even for several hours. The absorbent core comprises an absorbent material typically in granular or powder form, called SAP (acronym for "Super Absorbent Powder"), capable of absorbing liquids up to 300 times its own weight and up to 60 times its own volume.

The bottom layer is made impermeable to body fluids to prevent leakage of the absorbed liquids from the absorbent core. Typically, the bottom layer is made impermeable by a plastic membrane.

The female sanitary napkin is configured to be applied to a user's undergarment by means of adhesive portions placed on the bottom layer.

To make an absorbent article, a multilayer tape is formed by superimposing a bottom layer tape, in the form of a continuous tape, a plurality of successively arranged absorbent cores, and a top layer tape, in the form of a continuous tape.

The top and bottom layer tapes are joined together by arranging the absorbent cores between them. Subsequently, the multilayer tape is cut around each absorbent core to make a plurality of absorbent articles. For each absorbent article semi-finished product, the cut top layer tape defines the top layer of the absorbent article, the cut bottom layer tape defines the bottom layer of the absorbent article, and an absorbent core remains interposed between them.

In markets that are more sensitive to environmental issues, regulators and consumers prefer biodegradable products.

Therefore, biodegradable female sanitary napkins have recently been developed that can be disposed of in toilets and dissolved in water in a time of the order of magnitude of several days, weeks or months. In these products, the two top and bottom layer tapes and the absorbent core are made of biodegradable materials.

In particular, the bottom layer tape may comprise a permeable, cellulose-based main substrate made impermeable to fluids by means of a polyvinyl alcohol substrate superimposed on the main substrate, on the side facing the absorbent cores. Polyvinyl alcohol is a material that is impermeable to body fluids for a time (during use of the female sanitary napkin) and subsequently soluble in water (when the female sanitary napkin is disposed of).

The Applicant found that the polyvinyl alcohol substrate of the bottom layer tape, which is typically very thin (e.g., a few hundredths of a millimetre), tends to absorb ambient moisture and curl, causing the entire bottom layer tape to curl accordingly, particularly in the transverse direction, before it is superimposed with the absorbent cores and the top layer tape to form the multilayer tape.

The Applicant deduced that the greater the unsupported path of the bottom layer tape comprising the polyvinyl alcohol substrate, the greater the curl.

The Applicant thus decided to place along the path of the bottom layer tape a plurality of members configured to prevent the aforementioned curling.

WO 2011/025032 A1 discloses a prior art example of an apparatus for producing an absorbent sanitary article.

The present invention therefore concerns, in a first aspect thereof, an apparatus for making an absorbent sanitary article according to claim 1.

In the present description and subsequent claims, the term "stretching members" means members that are specially provided for stretching the lower tape transversely to the advancement direction and in the absence of which curling of the lower tape would occur.

In this description and subsequent claims, the expressions "transversely to the advancement direction" and "transverse to the advancement direction" mean along an inclined direction with respect to the advancement direction, preferably substantially perpendicular to the advancement direction.

In a second aspect, the present invention concerns a method for making an absorbent sanitary article according to claim 4.

The Applicant has found that by supporting and stretching the bottom layer tape transversely to the advancement direction by means of the aforementioned plurality of stretching members, it is possible to prevent curling of the bottom layer tape between the supply assembly and the forming assembly. These stretching members define additional zones of support for the bottom layer tape along the advancement direction of the latter and, consequently, allow the distance travelled by the bottom layer tape without support to be reduced, preventing curling.

The apparatus of the first aspect of the present invention enables the method of the second aspect of the present invention to be implemented.

The present invention may have, in both aspects discussed above, at least one of the preferred features described below. Such features may therefore be present individually or in combination, except where expressly stated otherwise, either in the apparatus of the first aspect of the present invention or in the method of the second aspect of the present invention.

Preferably, said bottom layer tape comprises a main substrate.

Preferably, said main substrate is made of biodegradable material, preferably cellulose-based.

Preferably, said polyvinyl alcohol substrate is laminated onto the main substrate.

In a preferred embodiment of the present invention, said supply assembly comprises a supply reel comprising a main substrate in the form of a wound tape and configured to unwind said main substrate.

In this case, preferably, said supply assembly comprises an applicator configured to laminate a polyvinyl alcohol substrate in tape form onto said main substrate unwound from said supply reel to obtain said bottom layer tape.

In the above preferred embodiment, preferably, supplying a bottom layer tape comprises unwinding a main substrate in tape form from a supply reel and subsequently laminating a polyvinyl alcohol substrate in tape form onto said main substrate unwound from said supply reel to obtain said bottom layer tape.

In an alternative embodiment of the present invention, said supply assembly comprises a supply reel comprising a bottom layer tape comprising said main substrate and said polyvinyl alcohol substrate wound together.

In such a case, preferably, said supply reel is configured to unwind said bottom layer tape to supply said bottom layer tape along said advancement direction.

In the above-mentioned alternative embodiment, preferably, supplying a bottom layer tape comprises unwinding said bottom layer tape from a supply reel.

The predetermined maximum value is the distance above which the polyvinyl alcohol substrate of the bottom layer tape begins to curl transversely noticeably.

Preferably, at least one of said stretching members comprises a rod or shaped plate oriented transversely with respect to said advancement direction.

Preferably, said rod or shaped plate comprises a contact surface configured to contact said bottom layer tape and having a concave or convex profile transverse to said advancement direction.

In an embodiment of the present invention, at least one of said stretching members comprises a roller.

Preferably, said roller comprises a convex or narrower side surface configured to be in contact with said bottom layer tape.

Preferably, said roller has an axis of rotation perpendicular to said advancement direction.

In an embodiment of the present invention, at least one of said stretching members comprises a pair of rollers facing each other.

The Applicant found that the transversely oriented rod or shaped plate generates less resistance to the passage of the bottom layer tape than the facing roller or pair of rollers, allowing a higher line speed.

Preferably, said top layer tape and/or said bottom layer tape and/or said absorbent core are made of biodegradable material. In this way, the multilayer tape of the absorbent sanitary article is, at least partially, biodegradable.

Further features and advantages of the present invention will become clearer from the following detailed description of a preferred embodiment thereof, with reference to the appended drawings and provided by way of indicative and non-limiting example, in which:
- Figure 1 is a schematic view of a part of an apparatus for making an absorbent sanitary article according to the present invention;
- Figures 1A, 1B and 1D are schematic views of details of the apparatus in Figure 1;
- Figure 1C is a schematic view of an alternative embodiment of a detail of the apparatus in Figure 1;
- Figure 2 is a schematic view of another part of the apparatus of the present invention;
- Figure 3 is an exploded perspective view of an absorbent sanitary article made with the apparatus of the present invention;
- Figure 4 is a schematic sectional view of the absorbent sanitary article in Figure 3.

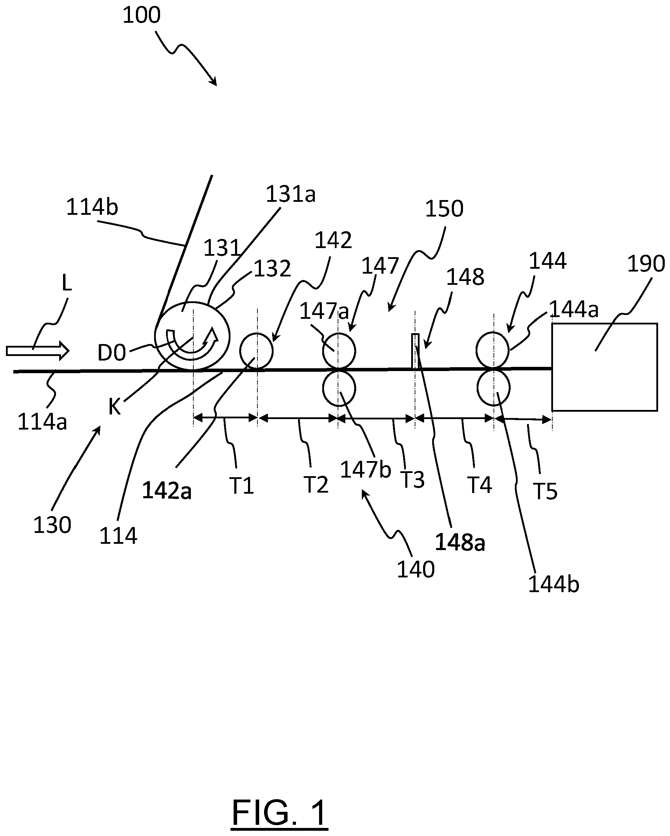

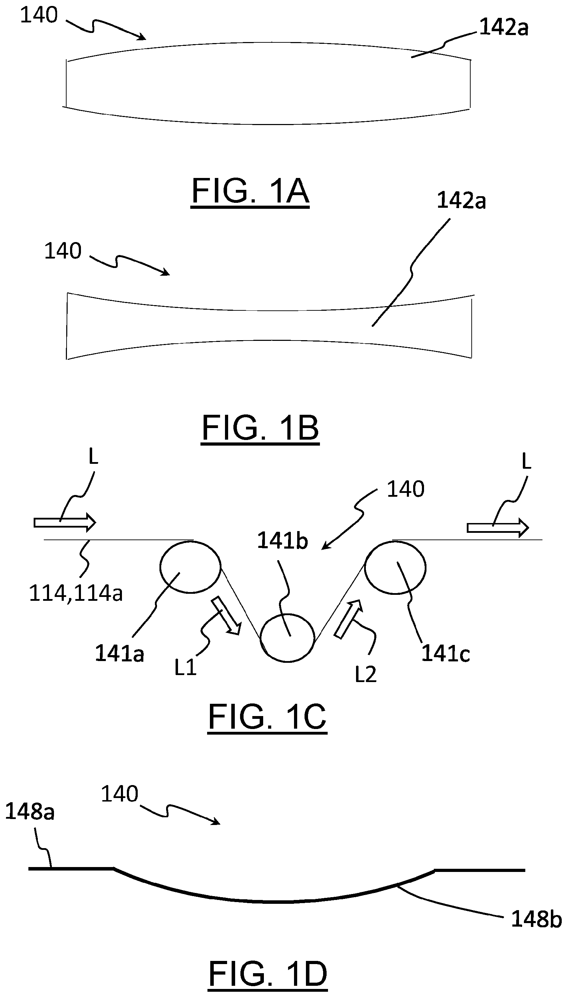

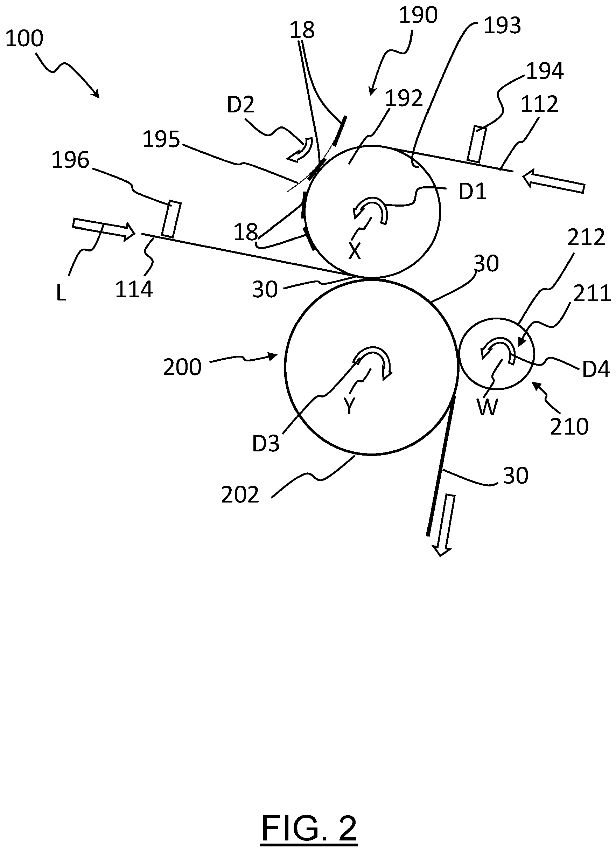

In Figures 1 and 2, the numerical reference 100 is used to indicate an apparatus for making an absorbent sanitary article in accordance with the present invention.

Non-limiting examples of absorbent sanitary articles made with the apparatus 100 are sanitary napkins for women, baby nappies, nappies for people suffering from incontinence and the like.

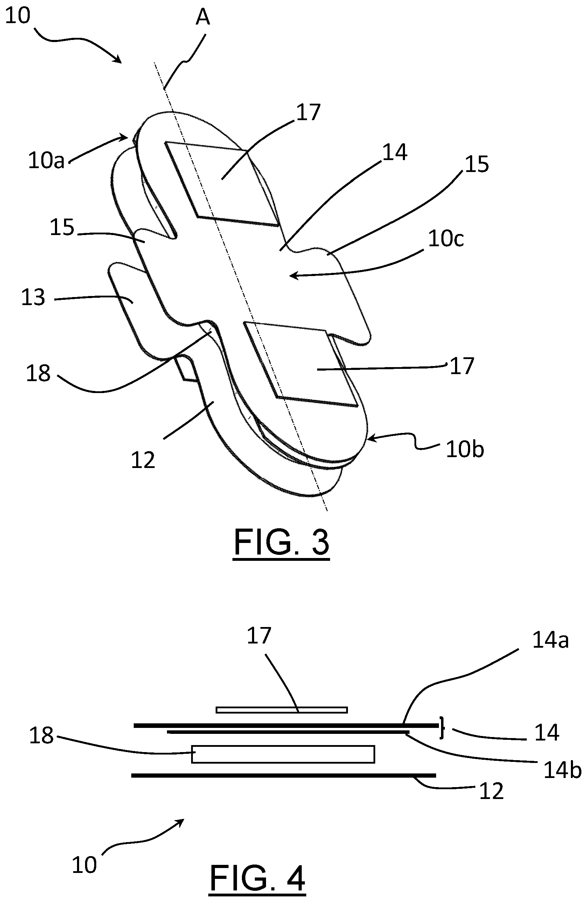

Without losing generality, explicit reference will be made below to a specific example of an absorbent sanitary article, such as the one illustrated in Figures 3 and 4 and indicated by numerical reference 10. It is specifically a female sanitary napkin. However, what is described could find similar application to different types of absorbent sanitary articles.

The article 10 has an elongated shape along a longitudinal axis A. With respect to the longitudinal axis A, the article 10 has a front part 10a, a rear part 10b and a central part 10c arranged between the front part 10a and the rear part 10b. The central part 10c is narrower than the front part 10a and the back part 10b.

The article 10 comprises a top layer 12, also known as the "topsheet", and a bottom layer 14, also known as the "backsheet", superimposed on each other. The top layer 12 and the bottom layer 14 define, respectively, an inner face and an outer face of the article 10. The top layer 12 and the bottom layer 14 extend longitudinally from the front part 10a to the back part 10b. The top layer 12 and the bottom layer 14 have the same shape and extension in plan as the shape and extension in plan of the article 10.

The top layer 12 is made of a biodegradable material permeable to body fluids, preferably cellulose fibres.

The bottom layer 14 comprises a main substrate 14a made of biodegradable material, preferably cellulose fibres, and a waterproofing substrate 14b for containing liquids (Figure 4). The waterproofing substrate 14b is configured to hold liquids for a predetermined time period, e.g. several hours, days or weeks, and to dissolve in water if immersed for longer than the predetermined time period. The waterproofing substrate 14b is made of polyvinyl alcohol.

Two fixing wings 13, 15 protrude from opposite sides of the article 10 transversely to the longitudinal axis A. The fixing wings 13, 15 protrude at the central part 10c, in particular at its narrower part. The fixing wings 13, 15 are formed from respective portions of the bottom layer 12 and the top layer 14. These portions are superimposed and directly in contact with each other.

The article 10 can be applied to an undergarment by means of adhesive portions 17 placed on the bottom layer 14. The adhesive portions 17 are arranged on the main substrate 14a opposite the waterproofing substrate 14b.

In use, the bottom layer 14 is turned towards the undergarment while the top layer 12 is turned away from the undergarment, towards the user's body.

An absorbent core 18 is placed between the top layer 12 and the bottom layer 14. This absorbent core 18 has the function of absorbing the body fluids that pass through the top layer 12 and retaining them without leaks even for several hours. The absorbent core 18 is made of absorbent material comprising a fibre matrix, e.g. cellulose, and an absorbent powder (SAP) dispersed in the fibre matrix, e.g. carboxymethyl cellulose (CMC). Thus, the absorbent core 18 is biodegradable. The absorbent core 18 extends longitudinally from the front part 10a to rear part 10b and is substantially oval in shape, narrower at the central part 10c.

Figures 1 and 2 show parts of the apparatus 100 that enables the manufacturing of the absorbent sanitary article 10.

The apparatus 100 comprises a forming assembly 190 configured to form a multilayer tape 30 of absorbent sanitary article 10. The forming assembly 190 is schematically illustrated in Figure 1 and is described in detail below with reference to Figure 2.

As illustrated in Figure 2, the multilayer tape 30 comprises a top layer tape 112, a bottom layer tape 114 and a plurality of absorbent cores 18 interposed successively between the top layer tape 112 and the bottom layer tape 114.

Still with reference to Figure 2, the forming assembly 190 comprises a forming drum 192 rotatable about the axis of rotation X. The forming drum 192 comprises an outer surface 193, preferably suctioned.

The forming drum 192 is configured to rotate in a direction D1 (which is anti-clockwise in Figure 2).

The apparatus 100 is configured to supply the top layer tape 112 onto the forming drum 192, in particular directly onto the outer surface 193. A first glue dispenser 194 is configured to dispense liquid glue, preferably water-based, onto the top layer tape 112 before the top layer tape 112 is supplied onto the forming drum 192, so as to impregnate it with glue. This glue is preferably biodegradable glue.

As shown in Figure 2, the forming assembly 190 includes a transport drum 195 (only partially shown) for transporting absorbent cores 18. The transport drum 195 is configured to hold a plurality of absorbent cores 18, preferably by suction, and to advance them in a direction D2 (which is clockwise in Figure 2), opposite to the direction D1 of the forming drum 192, by rotating about its axis of rotation (not illustrated).

The transport drum 195 is substantially tangential to the forming drum 192 and is configured to deposit the absorbent cores 18 onto the forming drum 192 by overlapping them with the top layer tape 112.

The apparatus 100 comprises a supply assembly 130, shown in Figure 1, configured to supply the bottom layer tape 114.

In the non-limiting example illustrated in Figure 1, the supply assembly 130 is configured to supply a main substrate 114a in tape form, e.g. by unwinding it from a supply reel (not illustrated). The main substrate 114a is made of a permeable biodegradable cellulose-based material. The supply assembly 130 comprises an applicator 131 configured to laminate a polyvinyl alcohol substrate 114b, in tape form, onto the main substrate 114a.

The main substrate 114a and the polyvinyl alcohol substrate 114b laminated onto it form the bottom layer tape 114 supplied by the supply assembly 130. In the illustrated embodiment of the present invention, the applicator 131 comprises a roller 131a rotatable about an axis of rotation K in a direction D0 (which is anticlockwise in Figure 1).

In a non-illustrated embodiment of the present invention, the supply assembly 130 is configured to supply the bottom layer tape 114 comprising the main substrate 114a and the polyvinyl alcohol substrate 114b already laminated onto it, for example by unwinding it directly from a supply reel.

The apparatus 100 is configured to advance the bottom layer tape 114, supplied by the supply assembly 130, along an advancement direction L to the forming assembly 190. The supply assembly 130 is therefore arranged upstream of the forming assembly 190 with respect to the advancement direction L of the bottom layer tape 114.

The bottom layer tape 114 is guided from the supply assembly 130 to the forming assembly 190 by a plurality of deflecting rollers (not illustrated), which define the advancement direction L. In Figure 1, the path of the bottom layer tape 114 from the supply assembly 130 to the forming assembly 190 is schematically depicted as straight and the advancement direction is one-directional, but this path can be deflected by the aforementioned deflecting rollers which change the advancement direction L as the bottom layer tape 114 advances.

Along the advancement direction L of the bottom layer tape 114, at a zone 150 arranged between the supply assembly 130 and the forming assembly 190, there are provided a plurality of stretching members 140 configured to contact the bottom layer tape 114 so as to support and stretch it transversely to the advancement direction L.

In the non-limiting example illustrated in Figure 1, the plurality of stretching members 140 comprise a first stretching member 142 placed downstream of the supply assembly 130, a last stretching member 144 placed upstream of the forming assembly 190 and downstream of the first stretching member 142, and two intermediate stretching members 147 and 148, placed between the first stretching member 142 and the last stretching member 144.

The first stretching member 142 is placed at a distance T1 from the supply assembly 130 that is lower than a predetermined maximum value. This predetermined maximum value is comprised between 200 mm and 1000 mm, preferably between 200 mm and 700 mm.

The intermediate stretching members 147, 148 are placed at a reciprocal distance T3 that is lower than this predetermined maximum value.

The intermediate stretching member 147 is placed at a distance T2 from the first stretching member 142 that is lower than this predetermined maximum value.

The intermediate stretching member 148 is placed at a distance T4 from the last stretching member 144 that is lower than this predetermined maximum value.

The last stretching member 144 is placed at a distance T5 from the forming assembly 190, which is lower than this predetermined maximum value.

The distances T1-T5 are measured along the advancement direction L of the bottom layer tape 114.

There can be more than two intermediate stretching members, so that in the zone 150 between the supply assembly 130 and the forming assembly 190 there are no sections in which the reciprocal distance between adjacent stretching members is greater than this predetermined maximum value.

In the case, not illustrated, in which the apparatus 100 comprises deflecting rollers between the supply assembly 130 and the forming assembly 190, if the distance between two successive deflecting rollers is greater than the predetermined maximum value, then one or more stretching members 140 are interposed between the two successive deflecting rollers such that the distance measured along the advancement direction L between each deflecting roller and the downstream or upstream deflecting member 140 is less than the predetermined maximum value.

For the purposes of the present description and the appended claims, the deflecting rollers are not considered to be stretching members 140 because their primary function is to deflect the bottom layer tape 114 and not to support and stretch the bottom layer tape 114.

Each stretching member 140 may comprise a roller or pair of rollers facing each other, or a rod or shaped plate oriented transversely to the advancement direction L of the bottom layer tape 114.

In the non-limiting example illustrated in Figure 1, the first stretching member 142 comprises a roller 142a, the intermediate stretching member 147 comprises a pair of facing rollers 147a, 147b, the intermediate stretching member 148 comprises a rod or shaped plate 148a oriented transversely with respect to the advancement direction L and the last stretching member 144 comprises a pair of facing rollers 144a, 144b.

Figure 1A shows an example of a stretching member 140 comprising a roller with a convex side surface 142a.

Figure 1B depicts a further example of a stretching member 140 comprising a roller with a narrower side surface 142a.

Figure 1C shows a further example of a stretching member 140, comprising three rollers 141a, 141b, 141c arranged in succession and offset from each other. The three rollers 141a, 141b, 141c comprise a first roller 141a configured to deflect the bottom layer tape 114 towards a direction L1 inclined with respect to the advancement direction L. A second roller 141b of the three rollers 141a, 141b, 141c is configured to deflect the bottom layer tape 114 towards a direction L2 inclined with respect to the advancement direction L on the opposite side with respect to the direction L1. A third roller 141c of the three rollers 141a, 141b, 141c is configured to deflect the bottom layer tape 114 towards an inclined direction with respect to the direction L2 such that the advancement direction L of the bottom layer tape 114 downstream of the third roller 141c is substantially equal to the advancement direction L of the bottom layer tape 114 upstream of the first roller 141a. The three rollers 141a, 141b, 141c are preferably spaced from each other, along the advancement direction L, by respective distances lower than the aforementioned predetermined maximum value.

Figure 1D depicts a further example of a stretching member 140, comprising a rod or shaped plate 148a comprising a contact surface 148b configured to contact the bottom layer tape 114 and having a concave or convex profile transversely to the advancement direction L.

Downstream of the stretching members 140, the bottom layer tape 114 is supplied to the forming assembly 190 along the advancement direction D. In particular, the bottom layer tape 114 is supplied onto the forming drum 192, superimposed with the top layer tape 112 and the absorbent cores 18 superimposed thereon. In this way, the absorbent cores 18 remain interposed between the top layer tape 112 and the bottom layer tape 114 and the multilayer tape 30 is formed.

A second glue dispenser 196 is configured to dispense liquid glue, preferably water-based, onto the bottom layer tape 114 before it is supplied onto the forming drum 192, in order to impregnate it with glue. This glue is preferably biodegradable glue.

In an alternative, non-illustrated embodiment of the present invention, only one of the first glue dispenser 194 and the second glue dispenser 196 could be provided so as to impregnate only one of the top layer tape 112 and the bottom layer tape 114.

A support drum 200 is adjacent to the forming drum 192, substantially tangential thereto. The support drum 200 is mounted rotatably on a frame 201, not illustrated. The support drum 200 comprises a support surface 202 defined on a cylindrical outer surface of the support drum 200. The support surface 202 is preferably smooth. In the remainder of this description, a surface is understood to be "smooth" if it is a uniform surface free of recesses, protrusions, knurls, holes, roughness and other obvious unevenness, and with a surface roughness lower than 100 µm, preferably lower than 10 µm.

The support drum 200 is rotatable about an axis of rotation Y. The axis of rotation Y is parallel to the axis of rotation X. The support drum 200 is configured to rotate in direction D3 (which is clockwise in Figure 2), opposite to direction D2.

The support surface 202 has a width greater than or equal to the width of the multilayer tape 30. The width of the support surface 202 is measured in the direction parallel to the axis of rotation Y of the support drum 200. The width of the multilayer tape 30 is measured in a direction orthogonal to a main longitudinal direction of the multilayer tape 30 and parallel to the axis of rotation Y of the support drum 200.

In the non-limiting example shown in Figure 2, the forming drum 192 is placed above the support drum 200. The diameter of the forming drum 192 is smaller than the diameter of the support drum 200.

The forming drum 192 is configured to supply the multilayer tape 30 onto the support drum 200. The multilayer tape 30 is advanced in direction D1 on the forming surface 193 of the forming drum 192 and passed between the forming drum 192 and the support drum 200. When the multilayer tape 30 arrives between the forming drum 192 and the support drum 200, the multilayer tape 30 is abandoned by the forming drum 192, e.g. by ceasing the suction action towards the forming surface 193, and taken over by the support drum 200.

The multilayer tape 30 is advanced on the support surface 202 in direction D3.

The apparatus 100 includes a second pressing member 210. In the non-limiting example illustrated in Figure 2, the second pressing member 210 comprises a compression roller 211 adjacent to the support drum 200 and configured to press the multilayer tape 30 against the support surface 202. The compression roller 211 has a cylindrical outer surface 212 that is preferably smooth.

The compression roller 211 is rotatably mounted on a support body (not shown), preferably in idle mode. The compression roller 211 is rotatable about an axis of rotation W parallel to the axis of rotation Y of the support drum 200. The compression roller 211 is configured to rotate about the axis of rotation W in a direction D4 (which is anticlockwise in Figure 2) opposite to direction D3 of the support drum 200.

The compression roller 211 is placed at a respective distance from the support surface 202. This distance defines a gap between the support drum 200 and the compression roller 211 through which the multilayer tape 30 is passed. This distance can be adjusted by means of special adjusting devices not illustrated.

The multilayer tape 30 is advanced on the support surface 202, in particular by rotation of the support drum 200, and passed between the support surface 202 and the compression roller 211, so as to be compressed between the two. The compression of the multilayer tape 30 improves the adhesion between the individual glue-impregnated layers of the multilayer tape 30.

In an alternative embodiment of the present invention not illustrated, there is provided an additional compression roller analogous to the compression roller 211 and arranged downstream of the compression roller 211 on the support surface 202.

The apparatus 100 is configured to allow the multilayer tape 30 to leave the support surface 202 downstream of the compression roller 211 (or the additional compression roller) and to be moved along an outlet path (not illustrated) for further processing (not illustrated).

In a final processing step (not illustrated), the multilayer tape 30 is cut into discrete elements each corresponding to a single absorbent article 10 so that from the top layer tape 112 is obtained the top layer 12 of the absorbent article 10 and bottom layer 14 of the absorbent article 10 is obtained from the bottom layer tape 114, with one of the absorbent cores 18 interposed between them.

Obviously, a person skilled in the art, in order to satisfy specific and contingent needs, can make numerous modifications and variations to the invention described above while remaining within the scope of protection defined by the following claims.

## Claims

1. Apparatus (100) for making an absorbent sanitary article (10), comprising:
a supply assembly (130) configured to supply a bottom layer tape (114) comprising a polyvinyl alcohol substrate (114b);
a forming assembly (190) arranged downstream of said supply assembly (130) along an advancement direction (L) of said bottom layer tape (114) and configured to form a multilayer tape (30) of absorbent sanitary article (10) comprising said bottom layer tape (114), a top layer tape (112) and a plurality of absorbent cores (18) arranged between said top layer tape (112) and said bottom layer tape (114);
a plurality of stretching members (140; 141a, 141b, 141c, 142, 144, 147, 148) arranged along said advancement direction (L) between said supply assembly (130) and said forming assembly (190) and configured to stretch said bottom layer tape (114) transversely to said advancement direction (L);
wherein said plurality of stretching members (140; 141a, 141b, 141c, 142, 144, 147, 148) comprises:
at least two intermediate stretching members (147, 148) placed at at least one reciprocal distance (T3) lower than a predetermined maximum value comprised between 200 mm and 1000 mm, preferably between 200 mm and 700 mm, said at least one reciprocal distance (T3) being measured along said advancement direction (L);
at least one first stretching member (142) placed upstream of said at least two intermediate stretching members (147, 148) at a first distance (T1) from said supply assembly (130) lower than a predetermined maximum value; and
at least one last stretching member (144) placed at a second distance (T5) from said forming assembly (190) lower than said predetermined maximum value, said first distance (T1) and second distance (T5) being measured along said advancement direction (L).

2. Apparatus (100) according to claim 1, wherein at least one (148) of said stretching members (140; 141a, 141b, 141c, 142, 144, 147, 148) comprises a rod or shaped plate (148a) oriented transversely with respect to said advancement direction (L).

3. Apparatus (100) according to claim 1 or 2, wherein at least one (142, 144, 147) of said stretching members (140; 141a, 141b, 141c, 142, 144, 147, 148) comprises a roller (142a) or a pair of rollers (147a, 147b; 144a, 144b) facing each other.

4. Method for making an absorbent sanitary article (10), comprising
supplying a bottom layer tape (114) comprising a polyvinyl alcohol substrate (114b) along an advancement direction (L) from a supply assembly (130) to a forming assembly (190) configured to form a multilayer tape (30) of absorbent sanitary article (10) comprising said bottom layer tape (114), an top layer tape (112) and a plurality of absorbent cores (18) interposed between said top layer tape (112) and said bottom layer tape (114);
stretching said bottom layer tape (114) transversely to said advancement direction (L) at a zone (150) arranged along said advancement direction (L) between said supply assembly (130) and said forming assembly (190);
wherein stretching said bottom layer tape (114) comprises:
placing said bottom layer tape (114) in contact with at least two intermediate stretching members (147, 148) placed at at least one reciprocal distance (T3) lower than a predetermined maximum value comprised between 200 mm and 1000 mm, preferably between 200 mm and 700 mm, said at least one reciprocal distance (T3) being measured along said advancement direction (L);
placing said bottom layer tape (114) in contact with at least one first stretching member (142) placed upstream of said at least two intermediate stretching members (147, 148) at a first distance (T1) from said supply assembly (130) lower than said predetermined maximum value;
placing said bottom layer tape (114) in contact with at least one last stretching member (144) placed downstream of said at least two intermediate stretching members (147, 148) at a second distance (T5) from said supply assembly (190) lower than said predetermined maximum value;
wherein said first distance (T1) and second distance (T5) are measured along said advancement direction (L).

## Patentansprüche

1. Vorrichtung (100) zur Herstellung eines absorbierenden Hygieneartikels (10), umfassend:
eine Zuführanordnung (130), die dazu konfiguriert ist, ein unteres Schichtband (114) zuzuführen, das ein Polyvinylalkohol-Substrat (114b) umfasst;
eine Formungsanordnung (190), die stromabwärts von der Zuführanordnung (130) entlang einer Vorschubrichtung (L) des Unterlagenbandes (114) angeordnet und so konfiguriert ist, dass sie ein mehrlagiges Band (30) eines absorbierenden Hygieneartikels (10) bildet, das das Unterlagenband (114), einem Oberschichtband (112) und einer Vielzahl von absorbierenden Kernen (18), die zwischen dem Oberschichtband (112) und dem Unterschichtband (114) angeordnet sind;
eine Vielzahl von Streckelementen (140; 141a, 141b, 141c, 142, 144, 147, 148), die entlang der Vorschubrichtung (L) zwischen der Zuführanordnung (130) und der Formungsanordnung (190) angeordnet und so konfiguriert sind, dass sie das untere Schichtband (114) quer zur Vorschubrichtung (L) dehnen;
wobei die Vielzahl von Streckelementen (140; 141a, 141b, 141c, 142, 144, 147, 148) Folgendes umfasst:
mindestens zwei Zwischenstreckelemente (147, 148), die in einer Distanz (T3) voneinander platziert sind, die kleiner ist als ein vorbestimmter Maximalwert zwischen 200 mm und 1000 mm, vorzugsweise zwischen 200 mm und 700 mm, wobei die Distanz (T3) entlang der Vorschubrichtung (L) gemessen wird;
mindestens ein erstes Streckelement (142), das stromaufwärts von den mindestens zwei Zwischenstreckelementen (147, 148) in einer ersten Distanz (T1) von der Zuführanordnung (130) platziert ist, die kleiner ist als ein vorbestimmter Maximalwert; und
mindestens ein letztes Streckelement (144), das in einer zweiten Distanz (T5) von der Formungsanordnung (190) platziert ist, der kleiner ist als der vorbestimmte Maximalwert, wobei der erste Abstand (T1) und der zweite Abstand (T5) entlang der Vorschubrichtung (L) gemessen werden.

2. Vorrichtung (100) nach Anspruch 1, wobei mindestens eines (148) der Streckelemente (140; 141a, 141b, 141c, 142, 144, 147, 148) eine Stange oder eine geformte Platte (148a) umfasst, die quer zur Vorschubrichtung ausgerichtet ist. (L) ausgerichtet ist.

3. Vorrichtung (100) nach Anspruch 1 oder 2, wobei mindestens eines (142, 144, 147) der
Streckelemente (140; 141a, 141b, 141c, 142, 144, 147, 148) eine Walze (142a) oder ein Paar von einander zugewandten Walzen (147a, 147b; 144a, 144b) umfasst.

4. Verfahren zur Herstellung eines absorbierenden Hygieneartikels (10), umfassend Zuführen eines unteren Schichtbandes (114), das ein Polyvinylalkohol-Substrat (114b) umfasst, entlang einer Vorschubrichtung (L) von einer Zuführanordnung (130) zu einer Formungsanordnung (190), die so konfiguriert ist, dass sie ein mehrschichtiges Band (30) eines absorbierenden Hygieneartikels (10) bildet, das das untere Schichtband (114), ein oberes Schichtband (112) und eine Vielzahl von absorbierenden Kernen (18), die zwischen dem oberen Schichtband (112) und dem unteren Schichtband (114) zwischengesetzt sind;
Strecken des unteren Schichtbandes (114) quer zu der Vorschubrichtung (L) in einem Bereich (150), der entlang der Vorschubrichtung (L) zwischen der Zuführanordnung (130) und der Formungsanordnung (190) angeordnet ist;
wobei das Strecken des unteren Schichtbandes (114) Folgendes umfasst:
Inkontaktbringen des unteren Schichtbandes (114) mit mindestens zwei Zwischenstreckelementen (147, 148), die in einer Distanz (T3) voneinander platziert sind, die kleiner ist als ein vorbestimmter Maximalwert zwischen 200 mm und 1000 mm, vorzugsweise zwischen 200 mm und 700 mm, wobei die Distanz (T3) entlang der Vorschubrichtung (L) gemessen wird;
Inkontaktbringen des unteren Schichtbandes (114) mit mindestens einem ersten Streckelement (142), das stromaufwärts von den mindestens zwei Zwischenstreckelementen (147, 148) in einer ersten Distanz (T1) von der Zuführanordnung (130) platziert ist, die kleiner ist als der vorbestimmte Maximalwert;
Inkontaktbringen des unteren Schichtbandes (114) mit mindestens einem letzten Streckelement (144), das stromabwärts von den mindestens zwei Zwischenstreckelementen (147, 148) in einer zweiten Distanz (T5) von der Zuführvorrichtung (190) platziert ist, die geringer ist als der vorbestimmte Maximalwert;
wobei die erste Distanz (T1) und die zweite Distanz (T5) entlang der Vorschubrichtung (L) gemessen werden.

## Revendications

1. Appareil (100) pour la fabrication d'un article sanitaire absorbant (10), comprenant :
un ensemble d'alimentation (130) configuré pour fournir un ruban de couche inférieure (114) comprenant un substrat d'alcool polyvinylique (114b) ;
un ensemble de formage (190) disposé en aval dudit ensemble d'alimentation (130) le long d'une direction d'avancement (L) dudit ruban de couche inférieure (114) et configuré pour former un ruban multicouche (30) d'article sanitaire absorbant (10) comprenant ledit ruban de couche inférieure (114), un ruban de couche supérieure (112) et une pluralité de noyaux absorbants (18) disposés entre ledit ruban de couche supérieure (112) et ledit ruban de couche inférieure (114) ;
une pluralité d'éléments d'étirement (140 ; 141a, 141b, 141c, 142, 144, 147, 148) disposés le long de ladite direction d'avancement (L) entre ledit ensemble d'alimentation (130) et ledit ensemble de formage (190) et configurés pour étirer ledit ruban de la couche inférieure (114) transversalement à ladite direction d'avancement (L) ;
dans lequel ladite pluralité d'éléments d'étirement (140 ; 141a, 141b, 141c, 142, 144, 147, 148) comprend :
au moins deux éléments d'étirement intermédiaires (147, 148) placés à au moins une distance réciproque (T3) inférieure à une valeur maximale prédéterminée comprise entre 200 mm et 1000 mm, de préférence entre 200 mm et 700 mm, ladite au moins une distance réciproque (T3) étant mesurée le long de ladite direction d'avancement (L) ;
au moins un premier élément d'étirement (142) placé en amont desdits au moins deux éléments d'étirement intermédiaires (147, 148) à une première distance (T1) dudit ensemble d'alimentation (130) inférieure à une valeur maximale prédéterminée ; et
au moins un dernier élément d'étirement (144) placé à une seconde distance (T5) dudit ensemble de formage (190) inférieure à ladite valeur maximale prédéterminée, ladite première distance (T1) et ladite seconde distance (T5) étant mesurées le long de ladite direction d'avancement (L).

2. Appareil (100) selon la revendication 1, dans lequel au moins un (148) desdits éléments d'étirement (140 ; 141a, 141b, 141c, 142, 144, 147, 148) comprend une tige ou une plaque profilée (148a) orientée transversalement par rapport à ladite direction d'avancement (L).

3. Appareil (100) selon la revendication 1 ou 2, dans lequel au moins un (142, 144, 147) desdits
éléments d'étirement (140; 141a, 141b, 141c, 142, 144, 147, 148) comprennent un rouleau (142a) ou une paire de rouleaux (147a, 147b ; 144a, 144b) se faisant face.

4. Procédé de fabrication d'un article sanitaire absorbant (10), comprenant
la fourniture d'un ruban de couche inférieure (114) comprenant un substrat d'alcool polyvinylique (114b) le long d'une direction d'avancement (L) à partir d'un ensemble d'alimentation (130) vers un ensemble de formage (190) configuré pour former un ruban multicouche (30) d'article sanitaire absorbant (10) comprenant ledit ruban de couche inférieure (114), un ruban de couche supérieure (112) et une pluralité de noyaux absorbants (18) interposés entre ledit ruban de couche supérieure (112) et ledit ruban de couche inférieure (114) ;
l'étirement dudit ruban de couche inférieure (114) transversalement à ladite direction d'avancement (L) dans une zone (150) disposée le long de ladite direction d'avancement (L) entre ledit ensemble d'alimentation (130) et ledit ensemble de formage (190) ;
dans lequel l'étirement dudit ruban de la couche inférieure (114) comprend :
le placement dudit ruban de couche inférieure (114) en contact avec au moins deux éléments d'étirement intermédiaires (147, 148) placés à au moins une distance réciproque (T3) inférieure à une valeur maximale prédéterminée comprise entre 200 mm et 1000 mm, de préférence entre 200 mm et 700 mm, ladite au moins une distance réciproque (T3) étant mesurée le long de ladite direction d'avancement (L) ;
le placement dudit ruban de la couche inférieure (114) en contact avec au moins un premier élément d'étirement (142) placé en amont desdits au moins deux éléments d'étirement intermédiaires (147, 148) à une première distance (T1) dudit ensemble d'alimentation (130) inférieure à ladite valeur maximale prédéterminée ;
le placement dudit ruban de la couche inférieure (114) en contact avec au moins un dernier élément d'étirement (144) placé en aval desdits au moins deux éléments d'étirement intermédiaires (147, 148) à une seconde distance (T5) dudit ensemble d'alimentation (190) inférieure à ladite valeur maximale prédéterminée ;
dans lequel la première distance (T1) et la seconde distance (T5) sont mesurées le long de la direction d'avancement (L).
